# EUROPÄISCHE PATENTSCHRIFT

(11) **EP 1 326 959 B1**
(45) Veröffentlichungstag und Bekanntmachung des Hinweises auf die Patenterteilung: **21.12.2005**
(21) Anmeldenummer: 01980429.3
(22) Anmeldetag: 24.09.2001
(51) Int. Cl.: C12M 1/00, C12M 1/04

(54) **BIOREAKTOR FÜR DIE KULTIVIERUNG VON MIKROORGANISMEN SOWIE VERFAHREN ZUR HERSTELLUNG DESSELBEN**
BIO-REACTOR FOR THE CULTIVATION OF MICRO-ORGANISMS AND METHOD FOR THE PRODUCTION THEREOF
BIOREACTEUR POUR LA CULTURE DE MICRO-ORGANISMES ET SON PROCEDE DE FABRICATION

(30) Priorität: 06.10.2000 DE 10049437
(43) Veröffentlichungstag der Anmeldung: 16.07.2003
(73) Patentinhaber: Fraunhofer-Gesellschaft zur Förderung der angewandten Forschung e.V., 80686 München (DE)
(72) Erfinder: TRÖSCH, Walter, 70329 Stuttgart (DE)
(74) Vertreter: Schrell, Andreas
(86) Internationale Anmeldenummer: PCT/EP2001/011022
(87) Internationale Veröffentlichungsnummer: WO 2002/031102

(56) Entgegenhaltungen:
- DE-A- 2 358 701
- FR-A- 1 307 047
- FR-A- 2 588 271
- GB-A- 291 146
- GB-A- 2 235 210
- US-A- 3 955 317

## Beschreibung

Die Erfindung betrifft einen Bioreaktor für die Kultivierung von Mikroorganismen. Ferner betrifft die Erfindung ein Verfahren zur Herstellung eines solchen Bioreaktors.

Bioreaktoren sind Fermenter, in denen biologische Stoffumwandlungen mit Enzymen, Mikroorganismen (Bakterien, Pilze, Hefen, Algen) sowie tierischen und pflanzlichen Zellen durchgeführt werden. Im Bioreaktor sollen jeweils prozessspezifisch die optimalen Bedingungen hinsichtlich Temperatur, pH-Wert und Nährstoffkonzentration hergestellt werden können. Zu den Aufgaben eines Bioreaktors gehören daher der Stofftransport innerhalb der Flüssigphase (Durchmischen), das Dispergieren einer zweiten Phase, meist Luft, um eine große Phasengrenzfläche für guten Stoffübergang zu erhalten (Zerteilen) und der Wärmetransport, um die erzeugte Wärme abzuführen. Die Bauform eines Bioreaktors hängt von seinem Einsatzbereich ab und muss dementsprechend die spezifischen Anforderungen des verwendeten biologischen Systems berücksichtigen.

Zur Kultivierung von phototrophen Mikroorganismen kommen sogenannte Photobioreaktoren zum Einsatz. Unter den in Frage kommenden Bauformern gilt der Airlift-Photobioreaktor als besonders geeignet, um phototrophe Mikroorganismen anzuzüchten und zu hoher Zelldichte zu führen. Der Airlift-Photobioreaktor weist häufig einen turmförmigen Reaktorkessel auf, bei dem durch Eintrag von Luft ein Flüssigkeitsumlauf innerhalb einer konstruktiv festgelegten Schlaufe erzeugt wird. Der Airlift-Bioreaktor ist dadurch in eine begaste und eine unbegaste Zone aufgeteilt, die boden- und kopfseitig miteinander verbunden sind, so dass sich aufgrund der hydrostatischen Druckdifferenz eine Pumpenwirkung einstellt, die zu einem Flüssigkeitsausstrom in der begasten Zone führt. Da die Durchmischung des Reaktormediums ausschließlich durch die Belüftung hervorgerufen wird, ist bei dieser Photobioreaktor-Bauform eine gute Durchmischung und ein hoher Gas-Flüssigkeits-Stoffaustausch bei geringem Energieeintrag erzielbar. Ein solcher Airlift-Photobioreaktor wird beispielsweise in der GB 2 235 210 oder in der DE 199 16 597 beschrieben.

Da zur Kultivierung von phototrophen Mikroorganismen eine Lichteinstrahlung hoher Intensität, insbesondere auch in die Tiefe des Reaktors, notwendig ist, weisen die Photobioreaktoren vorzugsweise ein großes Oberflächen-Volumen-Verhältnis auf und erfordern daher einen relativ hohen Materialeinsatz. Die Herstellung eines solchen Photobioreaktors ist unter anderem aufgrund der verwendeten Materialien wie Glas oder Plexiglas verhältnismäßig kompliziert, aufwendig und kostenintensiv.

Der Erfindung liegt daher die Aufgabe zu Grunde, einen kostengünstigen Bioreaktor für die Kultivierung von phototrophen Organismen, insbesondere Mikroorganismen, zu schaffen, der einfach, schnell, preiswert und zuverlässig herstellbar ist. Weiterhin soll ein geeignetes Verfahren zu dessen Herstellung angegeben werden. Darüber hinaus soll ein kostengünstiges Verfahren zur Kultivierung von Mikroorganismen angegeben werden.

Diese Aufgabe wird mittels eines Bioreaktors mit den Merkmalen des Anspruchs 1 gelöst. Der erfindungsgemäße Bioreaktor weist ein Gehäuse auf, das aus einem lichtdurchlässigen Material gebildet ist. Das Gehäuse ist vorzugsweise länger als breit ausgebildet und wird vorzugsweise in Längsrichtung, zum Beispiel von Gasblasen, durchströmt. Die Gasblasen, vorzugsweise Luftblasen, werden durch Gaseintrag in eine Flüssigkeit erzeugt, die in dem Bioreaktor enthalten ist. In dem Gehäuse ist eine Strömungsleiteinrichtung angeordnet, die mindestens zwei Septen umfasst, die quer zur Strömungsrichtung angeordnet sind. Als Septen werden im Rahmen der vorliegenden Erfindung flächige Gebilde bezeichnet, die das Gehäuse in Unterkammern unterteilen, die miteinander in Verbindung stehen. Die Septen dienen dazu, die Strömung auf einem bestimmten Strömungsweg durch das Gehäuse des Bioreaktors zu leiten.

Vorzugsweise sind die Septen so ausgebildet und in dem Gehäuse angeordnet, dass sich zwischen den Septen in dem Gehäuse für die Gasblasen ein im Wesentlichen mäanderförmiger Strömungsweg ergibt. Die sich bewegenden Gasblasen versetzen die zwischen zwei aufeinanderfolgenden Septen befindliche Flüssigkeit in eine walzenförmige Bewegung. Durch die Anordnung der Septen wird erreicht, dass sich der Drehsinn der walzenförmigen Bewegung von Unterkammer zu Unterkammer umkehrt. Das führt zu der Ausbildung einer turbulenten Strömung und einer guten Durchmischung der in dem Bioreaktor enthaltenen Organismen.

Vorteilhafterweise sind die Septen so ausgebildet und in dem Gehäuse angeordnet, dass sie den durchströmten Querschnitt jeweils auf einen Spalt begrenzen. Die von den Septen begrenzten Spalte sind alternierend an gegenüberliegenden Seitenwänden des Gehäuses angeordnet. Im Betrieb ist in dem Bioreaktor eine Flüssigkeit enthalten, in die Gas eingetragen wird. Die aufgrund des Gaseintrags gebildeten Gasblasen führen beim Durchströmen der Spalte zur Ausbildung einer walzenförmigen Strömung in den von den Spalten im Gehäuse gebildeten Unterkammern.

Vorzugsweise sind die Septen durch mindestens ein Tragelement miteinander verbunden. Durch das Tragelement werden die Septen in ihrer Lage relativ zueinander und in dem Gehäuse fixiert. Bei dem Tragelement kann es sich zum Beispiel um einen in Strömungsrichtung verlaufenden schmalen Steg handeln, der die Strömung in dem Gehäuse praktisch nicht beeinflusst. Die Septen können auch an dem Gehäuse befestigt oder einstückig mit dem Gehäuse ausgebildet sein.

Vorzugsweise umfasst das Gehäuse zwei, insbesondere baugleiche Grundkörper jeweils mit einer lichten Tiefe T₃ gleich der halben lichten Tiefe T des Bioreaktors, wobei der Grundkörper wannenförmig aus einem Bodenteil und vier auf dem Bodenteil angeordneten, eine lichte Tiefe T₁ aufweisenden Seitenteilen aufgebaut ist. Der Grundkörper besteht aus einem lichtdurchlässigen Material und die baugleichen Grundkörper sind deckungsgleich aufeinander angeordnet. Im Inneren der baugleichen Grundkörper ist die Strömungsleiteinrichtung angeordnet. Der erfindungsgemäße Bioreaktor ist unter anderem aufgrund dieser Merkmale, insbesondere durch seine Ausgestaltung aus zwei bauidentischen Grundkörpern, in einfacher Weise sowie schnell, kostengünstig und zuverlässig herstellbar. Bei der Strömungsleiteinrichtung kann es sich um ein separates Bauteil handeln. Die Strömungsleiteinrichtung kann aber auch einstückig mit den Grundkörpern ausgebildet sein.

Eine besondere Ausführungsart der Erfindung ist dadurch gekennzeichnet, dass die Strömungsleiteinrichtung mindestens ein Septum, vorzugsweise mehrere Septen, umfasst, durch das beziehungsweise die zwei als Längsseitenteile bezeichnete Seitenteile, die parallel gegenüberliegend im rechten Winkel zu dem Septum beziehungsweise den Septen angeordnet sind, so miteinander verbunden sind, dass in dem wannenförmigen Grundkörper mehrere auf der von dem zugehörigen Bodenteil abgewandten Seite offene Kammern gebildet werden, wobei das mindestens eine Septum, eine Septumtiefe T₂ kleiner als die oder gleich der lichten Tiefe T des Reaktors und größer als die halbe lichte Tiefe T des Reaktors aufweist, wobei die Distanzen D zwischen den einzelnen Septen gleich sind und die Distanz D₁ zwischen einem als erstes Querseitenteil bezeichneten Seitenteil und einem darauf folgenden Septum beziehungsweise dem unmittelbar benachbarten Septum dieses Grundkörpers ungleich der Distanz D₂ zwischen einem als zweites Querseitenteil bezeichneten Seitenteil und einem darauf folgenden Septum beziehungsweise dem unmittelbar benachbarten Septum dieses Grundkörpers oder einem ganzzeiligen vielfachen davon ist, wobei die baugleichen Grundkörper mit ihren Kammeröffnungen gegenüberliegend so aufeinander angeordnet sind, dass die Distanz D₂ über der ersten Distanz D₁ aufliegt. Durch die Integration der Strömungsleiteinrichtung in die Grundkörper wird die Herstellung des erfindungsgemäßen Bioreaktors erheblich vereinfacht.

Vorzugsweise wird vorgesehen, dass die beiden bauidentischen Grundkörper in entgegengesetzter Orientierung aufeinander gelegt und miteinander verklebt, verschweißt oder sonst wie verbunden werden. Die spezielle Geometrie der Septen und insbesondere ihrer Distanzen zueinander und zu den Querseitenteilen führt dazu, dass der von den beiden Grundkörpern gebildete Reaktorraum in viele verschiedene durch die Septen teilweise voneinander abgetrennte Unterkammern unterteilt wird und wobei die Septen jeweils abwechselnd, gesehen über die Längsachse des gebildeten Bioreaktors, von dem einen und dem anderen Grundkörper ausgebildet sind. Die Septen der beiden Grundkörper bilden alternierend einmal von einem Bodenteil ausgehend, zum Beispiel von oben, und von dem anderen Bodenteil ausgehend, zum Beispiel von unten, also Unterkammern, durch die das Reaktormedium strömen kann. Vorzugsweise ist vorgesehen, dass die Septen in ihrer lichten Tiefe T₂ kleiner, vorzugsweise etwas kleiner oder aber auch gleich der lichten Tiefe T des Reaktors sind. In Letztgenannter Ausführungsform schließen die Septen die Unterkammern also vollständig voneinander ab. Aufgrund der flexiblen Materialstruktur kann die Strömung die Septen jedoch zur Seite biegen, so dass ein Flüssigkeitsstrom vom Reaktoreinlass durch die Unterkammern bis zum Reaktorauslass möglich wird. In einer weiteren Ausführungsform ist die lichte Tiefe T₂ der Septen kleiner als die lichte Tiefe T des Reaktors, so dass ein Spalt verbleibt, durch den die Flüssigkeit ebenfalls, unter Bildung von Turbulenzen, durchströmen kann.

Erfindungsgemäß wird unter dem Begriff wannenförmig ein von fünf Rechtecken begrenzter Grundkörper verstanden, der aus einem Bodenteil und vier Seitenteilen gebildet wird und bei dem die dem Bodenteil gegenüberliegende Fläche offen bleibt, das heißt nicht durch ein zweites Bodenteil abgedeckt ist. Die Ecken des Grundkörpers können hierbei entweder kantig oder abgerundet ausgebildet sein.

Die Septen eines Grundkörpers sind in ihrer lichten Tiefe T₂ derart ausgebildet, dass sie im erfindungsgemäßen Bioreaktor entweder das Bodenteil des gegenüberliegenden Grundkörpers gerade berühren oder einen Spalt zwischen Septumoberkante und Bodenteil bilden, damit ein im Reaktor befindliches Medium an dieser Stelle am Septum unter Bildung von Turbulenzen vorbeifließen kann. Es ist vorgesehen, dass die lichte Tiefe T₂ der Septen 60, 70, 80, 90, 95, 96, 97, 98 und vorzugsweise 99% der lichten Tiefe T des Bioreaktors aufweist. Unter der lichten Tiefe T₂ des Septums oder der lichten Tiefe T₁ der Seitenteile (gleich der lichten Tiefe T₃ der Grundkörper) wird die Tiefe dieser Bauteile unter Abzug der Materialdicke des Bodenteils verstanden.

Unter der lichten Tiefe T des Bioreaktors wird die Tiefe des Bioreaktors unter Abzug der Materialdicke der beiden Bodenteile der Grundkörper verstanden, die den Bioreaktor konstituieren. Durch die Ausgestaltung des Bioreaktors mit distanziert zueinander und jeweils alternierend an den Bodenteilen der beiden gegenüberliegenden Grundkörper ausgebildeten Septen wird der Reaktorraum des erfindungsgemäßen Bioreaktors als sogenannte Wirbelzellenkolonne ausgestaltet. Die Septen dienen hierbei der Erzeugung einer turbulenten Strömung des im Bioreaktor befindlichen Mediums in der Weise, dass sie den Strömungsquerschnitt erheblich verkleinern und somit als Drosselstelle wirken, mit der Folge einer Wirbelbildung und intensiven Durchmischung. Die erfindungsgemäßen Bioreaktoren eignen sich daher besonders für die Kultivierung phototropher Organismen, wie Mikroorganismen oder Pflanzen, zum Beispiel Algen.

Eine weitere besondere Ausführungsart der Erfindung ist dadurch gekennzeichnet, dass der Grundkörper in Längsrichtung mit mindestens einer Verstärkung ausgestattet ist. Die auf den Grundkörper bezogene Längsrichtung wird durch die Anordnung der Längsseitenteile definiert. Entsprechend wird die Querrichtung durch die Anordnung der Querseitenteile des Grundkörpers festgelegt. In Querrichtung sorgen die Septen für eine ausreichende Stabilität des Grundkörpers. Durch die zusätzliche Längsverstärkung wird eine ausreichende Stabilität auch in Längsrichtung des Grundkörpers gewährleistet. Dadurch wird eine unerwünschte Verformung des Bioreaktors in Betrieb verhindert.

Eine weitere besondere Ausführungsart der Erfindung ist dadurch gekennzeichnet, dass die Verstärkung von mehreren U-förmigen Unterzügen gebildet wird, die jeweils mit zwei benachbarten Septen und dazwischen mit dem Bodenteil des zugehörigen Grundkörpers verbunden sind, wobei das erste und das zweite Querseitenteil ebenfalls über jeweils einen U-förmigen Unterzug mit dem darauf folgenden Septum verbunden sind. Dadurch wird auf einfache Art und Weise eine Versteifung des Grundkörpers zwischen dem ersten und dem zweiten Querseitenteil erreicht. Die U-förmigen Unterzüge sind vorzugsweise einstückig mit dem zugehörigen Grundkörper. Dadurch vereinfacht sich die Herstellung des erfindungsgemäßen Bioreaktors. Außerdem haben die U-förmigen Unterzüge im Wesentlichen die gleiche Dicke wie die Septen. Dadurch wird erreicht, dass die Strömung in Längsrichtung durch den erfindungsgemäßen Bioreaktor nicht beeinträchtigt wird. Alternativ zu den einzelnen Unterzügen kann auch ein durchgehender Unterzug vorgesehen sein, der sich zwischen den beiden Querseitenteilen eines Grundkörpers erstreckt. Zusätzlich oder alternativ zu den Unterzügen können auch Verstrebungen auf der Außenseite des Grundkörpers vorgesehen sein. Darüber hinaus kann in das Material des Grundkörpers ein Matrix-Werkstoff, zum Beispiel in Form einer Fasermatte, eingebracht werde, um die Stabilität des Grundkörpers zu erhöhen.

Eine weitere besondere Ausführungsart der Erfindung ist dadurch gekennzeichnet, dass die Wandstärke der Septen zu dem Bodenteil des zugehörigen Grundkörpers hin zunimmt. Dadurch wird die Stabilität der Septen bei gleichzeitiger Materialeinsparung erhöht. Außerdem wird eine einfache Entformbarkeit der Septen gewährleistet, wenn der Grundkörper zum Beispiel im Spritzgießverfahren hergestellt wird.

Eine vorteilhafte Ausgestaltung des erfindungsgemäßen Bioreaktors sieht vor, dass das lichtdurchlässige Material, aus dem der Grundkörper besteht oder der wesentlicher Bestandteil des Grundkörpers ist, eine flexible Folie ist, vorzugsweise aus Kunststoff wie beispielsweise Polypropylen, Polystyrol oder Polyethylen. Die Verwendung eines solchen Reaktormaterials bietet erhebliche Kostenvorteile gegenüber den bisher verwendeten Materialien aus Glas oder Plexiglas. Die Verwendung einer flexiblen Folie ist insbesondere aufgrund der vorstehend genannten geometrischen Merkmale des Grundkörpers möglich. Der aus der flexiblen Folie bestehende Reaktorraum hält dadurch zum einen dem sich bildenden hydrostatischen Druck stand, zum anderen wird durch Gaseintrag an der Unterseite des Reaktors eine hohe Wirbel- bzw. Walzenausbildung in der Strömung des Reaktormediums gewährleistet, was die Voraussetzung für das Erzielen einer hohen Photosyntheseaktivität ist.

Zur Erzielung einer angemessenen Steifigkeit ist es vorteilhaft, wenn in bevorzugter Ausführung die Wandstärke der Seitenteile des Grundkörpers größer als die Wandstärke der Septen des Grundkörpers ist.

Eine vorteilhafte Ausgestaltung des Bioreaktors ist ferner, dass seine beiden Grundkörper an den zum Bodenteil beabstandeten Kanten der Seitenteile einen im wesentlichen rechtwinklig nach außen abstehenden Rand oder Kragen aufweisen. Dadurch ergibt sich ein gesicherter und definierter Anlagekontakt, an dem die beiden Grundkörper miteinander beispielsweise mittels Klebung oder mittels Ultraschall miteinander verbindbar sind. Eine vergrößerte Kontaktfläche erhöht ferner die Biegesteifigkeit des Bioreaktors.

Nach einer Weiterbildung des Bioreaktors ist vorgesehen, dass der von beiden Grundkörpern gebildete Reaktorraum eine Oberflächenvergrößerung größer als eine geradflächige umhüllende Fläche seines Volumens aufweist. Eine solche Oberflächenvergrößerung wird bereits durch das Vorhandensein der Septen hervorgerufen. Weiterhin besteht die Möglichkeit, die Reaktoroberfläche mäanderförmig oder sinusförmig auszubilden. Möglich ist aber auch, dass zur Oberflächenvergrößerung die Umhüllende des Bioreaktors Fortsätze beziehungsweise Wölbungen aufweist. Die Oberflächenvergrößerung führt zu einer besseren räumlichen Verteilung des Lichts über den Reaktorquerschnitt und damit zu einer Optimierung der Lichtintensität im gesamten Reaktor. Die Maßnahmen zur Oberflächenvergrößerung wirken zusätzlich turbolenzerhöhend auf die Strömungsführung des Reaktormediums. Wie bereits vorstehend erwähnt ist eine turbolente Strömungsführung mit Wirbel- beziehungsweise Walzenausbildung anzustreben, da dadurch die einstrahlende Lichtmenge und infolgedessen die Photosyntheseaktivität erhöht werden kann. Um eine maximale Photosyntheseaktivität zu erreichen, ist beispielsweise eine Lichteinstrahlfrequenz von mindestens einem Hertz zu ermöglichen (Flashing-Light-Effect).

Eine weitere vorteilhafte Ausgestaltung des Bioreaktors ist, dass dieser Elemente aufweist, die Licht von außen in den Reaktorraum leiten, um die Energiedichte im Reaktorraum zu erhöhen. Dies geschieht beispielsweise durch sogenannte Wellenlängenschieber. Durch Wellenlängenschieber wird der durch phototrophe Mikroorganismen nicht absorbierbare Anteil des Lichtes so konvertiert, dass ein möglichst großer Lichtanteil oder die Gesamtheit der Strahlung in jenes Frequenzband verschoben werden kann, das von dem Photozentrum des eingesetzten phototrophen Mikroorganismus absorbierbar ist. Somit wird die holometrische Strahlungsdichte spezifisch so erhöht, dass pro Reaktorvolumen gegenüber mit Normallicht bestrahlten Bioreaktoren die Produktivität wesentlich erhöht wird. Eine Wellenlängenverschiebung kann beispielsweise dadurch erzielt werden, dass eine wellenlängenverschiebende Substanz als Anstrich auf der Innenseite oder Außenseite der Reaktorwand aufgetragen wird. Ebenso ist eine Wellenlängenverschiebung durch Stäbe, Platten, Fasern oder Partikel im Reaktorraum möglich.

Es ist vorteilhafterweise vorgesehen, dass der Bioreaktor Anschlüsse und Anschlussleitungen für die Zu- und/oder Abfuhr von Gasen und/oder Flüssigkeiten aufweist. Dadurch kann ein Austausch der Betriebsstoffe vollautomatisch mittels einer Steuereinrichtung durchgeführt werden. Ferner ist vorgesehen, dass die Anschlussleitungen kühl- und/oder heizbar sind. Dies ergibt vortei-lhafterweise zudem die Möglichkeit, dass sowohl die zugeführten, als auch die abgeführten Stoffe auf einer definierten Temperatur gehalten werden können. Die Kühlung oder Heizung kann durch interne oder externe Schlaufen erfolgen. Die Überwachung und Regelung einer solchen Kühlung beziehungsweise Heizung kann wiederum mittels der Überwachungs- beziehungsweise Steuereinrichtung erfolgen. Der erfindungsgemäße Bioreaktor wird vorzugsweise so aufgestellt, dass die Längsseitenteile der beiden den Bioreaktor bildenden Grundkörper vertikal angeordnet sind. Bei dieser Anordnung des Bioreaktors ist jeweils ein Querseitenteil der beiden Grundkörper nach unten und jeweils ein Querseitenteil nach oben gewandt. Vorzugsweise ist in einem der beiden nach unten gewandten Querseitenteile ein Gaseinlass vorgesehen. Durch den Gaseinlass kann Gas in das Reaktormedium eingetragen werden. Das Gas bildet in dem Reaktormedium Blasen, die nach oben steigen. Bei dem Gas handelt es sich zum Beispiel um Luft, die an einem Gasauslass austreten kann, der in einem der nach oben gewandten Querseitenteile der beiden Grundkörper vorgesehen sein kann. Durch den Gaseintrag in den erfindungsgemäßen Bioreaktor kommt es zur Ausbildung einer walzenförmigen, turbulenten Strömung in den Unterkammern des Bioreaktors. Das als Antrieb verwendete Gas wird vorzugsweise mit CO₂ angereichert, um das Wachstum der im Bioreaktor enthaltenen Mikroorganismen zu beschleunigen. Eine Begrenzung des Wachstums kann dadurch erfolgen, dass der Lichteinfall in den Bioreaktor limitiert wird. Darüber hinaus können an dem erfindungsgemäßen Bioreaktor Flüssigkeitsrückführleitungen vorgesehen sein, die von oben nach unten verlaufen. Die Flüssigkeitsrückführleitungen dienen dazu, die Durchmischung des Reaktormediums zu verbessern. Die Flüssigkeitsrückführleitungen können außerhalb des Bioreaktors verlaufen und gekühlt ausgebildet sein.

Nach einer Weiterbildung des Bioreaktors ist vorgesehen, dass der Bioreaktor Bestandteil eines Systems aus mehreren Einzel-Bioreaktoren darstellt, die fluidseitig in Reihe geschaltet sind. Mit einer solchen Ausgestaltung ist es möglich, die Leistung des Bioreaktors zu erhöhen. Die Erfindung betrifft daher auch Systeme, die aus in Reihe oder/und parallel zueinander geschalteten einzelnen Bioreaktoren der vorliegenden Erfindung aufgebaut sind.

Die Aufgabe wird ferner mittels eines Verfahrens zur Herstellung eines Bioreaktors gemäß Anspruch 1 gelöst, das die Merkmale des Anspruchs 16 aufweist. Bei diesem Verfahren wird in einem ersten Verfahrensschritt aus einem lichtdurchlässigen Material ein Strang hergestellt, der eine Vielzahl von direkt aufeinander folgenden, in Reihe angeordneten Grundkörpern aufweist, wobei die Grundkörper jeweils in der Orientierung D₂ eines Grundkörpers gefolgt von D₁ eines weiteren Grundkörper hintereinander angeordnet sind. Die Herstellung dieses Stranges erfolgt vorzugsweise in einem Spritzverfahren. In einem zweiten Verfahrensschritt wird der Strang in die einzelnen Grundkörper zerlegt und in einem dritten Verfahrensschritt werden jeweils zwei aufeinanderfolgende Grundkörper durch Umklappen eines zweiten auf einen ersten Grundkörper deckungsgleich und mit den Kammeröffnungen gegenüberliegend aufeinander angeordnet, so dass die Distanz D₂ über einer Distanz D₁ aufliegt. Anschließend oder gleichzeitig werden die beiden Grundkörper an dem jeweils nach außen abstehenden Kragen der vom Bodenteil beabstandeten Kante der Seitenteile des Grundkörpers miteinander verklebt oder mittels Ultraschall miteinander verschweißt. Das Verfahren ermöglicht die Herstellung eines Bioreaktors in einfacher und schneller Art und Weise. Dadurch, dass die vorgenannten Verfahrensschritte vollautomatisch durchgeführt werden können, wird ferner ein hohes Maß an Zuverlässigkeit bei der Herstellung des Bioreaktors erzielt.

Die Aufgabe wird des weiteren durch ein Verfahren zur Kultivierung von Mikroorganismen gelöst, das die Merkmale des Anspruchs 23 aufweist. Die Mikroorganismen werden in einen mit einer Flüssigkeit gefüllten Bioreaktor nach einem der Ansprüche 1 bis 19 eingebracht und dort unter geeigneten Bedingungen kultiviert. Vorzugsweise erfolgt die Kultivierung unter Gaseintrag..Durch den Gaseintrag bilden sich in der Flüssigkeit Gasblasen, die zu einer guten Durchmischung der Mikroorganismen in dem Bioreaktor beitragen. Es ist vorteilhaft, wenn die Kultivierung unter Zufuhr von CO₂ stattfindet. Das zugeführte CO₂ dient dazu, das Wachstum der Mikroorganismen in dem Bioreaktor zu beschleunigen. Vorzugsweise findet die Kultivierung unter Kühlung statt. Die Kühlung kann durch externe oder interne Kühlschlaufen erfolgen.

Weitere vorteilhafte Ausgestaltungen ergeben sich aus den Unteransprüchen.

Die folgenden Zeichnungen veranschaulichen die Erfindung anhand verschiedener Ausführungsformen. Es zeigt:
- Figur 1: eine Seitenansicht des erfindungsgemäßen Bioreaktors als Schnittdarstellung,

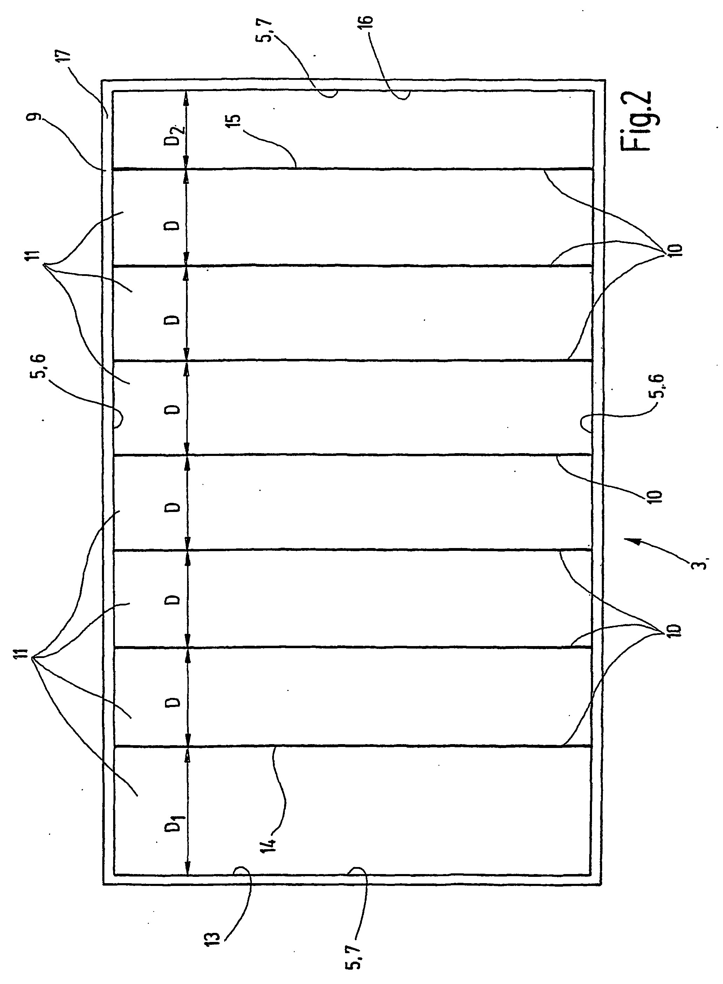

- Figur 2: eine Draufsicht auf den untenliegenden Grundkörper des Bioreaktors, und
- Figur 3: einen vergrößerten Ausschnitt aus Figur 1 gemäß einer weiteren Ausführungsform der Erfindung mit Längsverstärkung.

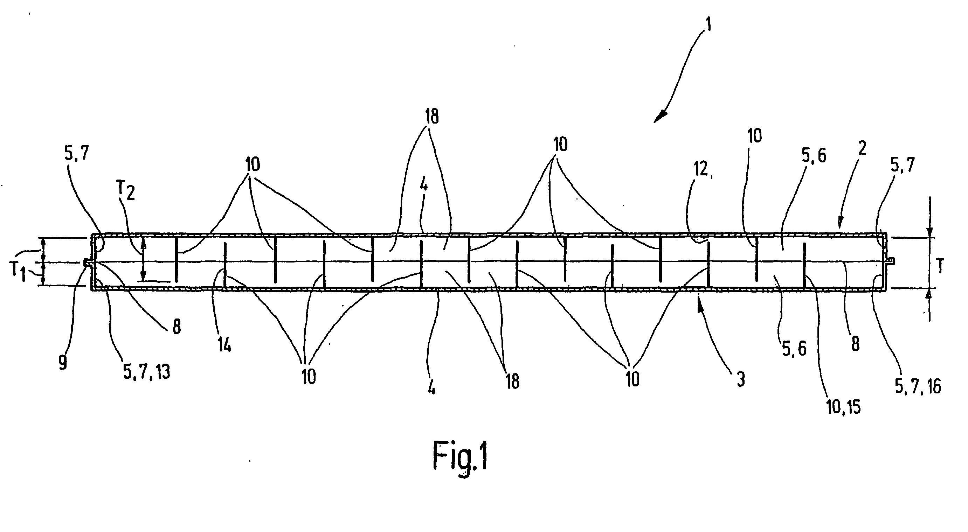

Die Figur 1 zeigt einen Bioreaktor 1, der aus zwei baugleichen rechteckigen Grundkörpern 2 und 3 aus einer dünnen, flexiblen, lichtdurchlässigen Kunststofffolie besteht. Jeder der beiden Grundkörper 2 und 3 ist einstückig aus einem Bodenteil 4 und auf dem Bodenteil 4 angeordneten Septen 10 sowie ebenfalls auf dem Bodenteil 4 angeordneten, eine lichte Tiefe T₁ aufweisenden Seitenteilen 5 aufgebaut, wobei jeweils zwei Seitenteile 5 als Längsseitenteile 6 sowie als Querseitenteile 7 ausgebildet sind. Die einander parallel und gegenüberliegend angeordneten Längsseitenteile 6 und die im rechten Winkel dazu ebenfalls parallel und gegenüberliegend angeordneten Querseitenteile 7 weisen an ihrer zum Bodenteil 4 beabstandeten Kante 8 einen rechtwinklig nach außen abstehenden Kragen 9 auf. Die beiden Längsseitenteile 6 eines Grundkörpers 2 beziehungsweise 3 sind durch sieben Septen 10 aus derselben flexiblen, dünnen Kunststofffolie derart miteinander verbunden, dass in dem Grundkörper 2 beziehungsweise 3 acht nach oben beziehungsweise nach unten hin offene rechtwinklige Kammern 11 gebildet werden. Die Septen 10 sind in gleichem Abstand jeweils in einer Distanz D in Längsrichtung zueinander angeordnet. Der Abstand zwischen einem ersten Querseitenteil 13 und einem ersten darauf folgenden Septum 14 wird als Distanz D₁ bezeichnet, die größer ist als die Distanz D zwischen den einzelnen Septen 10. Dagegen ist die Distanz D₂ zwischen dem letzten Septum 15 und dem zweiten Querseitenteil 16 kleiner als die Distanz D zwischen den Septen 10. Die Septen weisen eine gleiche lichte Tiefe T₂ auf, die kleiner als die zweifache lichte Tiefe T₁ des Grundkörpers 2 beziehungsweise 3 aber größer als die lichte Tiefe T₁ eines Grundkörpers 2 oder 3 ist. Dadurch ergibt sich im zusammengebauten Zustand der beiden den Bioreaktor 1 bildenden Grundkörper 2 und 3 ein Spalt 12 zwischen den oberen Kanten der Septen 10 des einen Grundkörpers 2 beziehungsweise 3 und dem Bodenteil 4 des gegenüberliegenden Grundkörpers 3 beziehungsweise 2. Die beiden baugleichen Grundkörper 2 und 3 sind deckungsgleich mit ihren Kammeröffnungen 11 gegenüberliegend angeordnet und zwar in der Weise, dass die Distanz D₂ eines Grundkörpers 2 beziehungsweise 3 über der ersten Distanz D₁ des anderen Grundkörpers 3 beziehungsweise 2 aufliegt, das heißt ihr direkt gegenüberliegend angeordnet ist. Die beiden baugleichen Grundkörper 2 und 3 sind also so aufeinander angeordnet, dass der obenliegende Grundkörper 2 umgeklappt, das heißt um 180° um seine Querachse gedreht, auf dem unten liegenden Grundkörper 3 aufliegt. Beide Grundkörper 2 und 3 berühren sich auf der durch den Kragen 8 der Seitenteile 4 gebildeten Kontaktfläche 17 und sind dort fluiddicht miteinander verbunden. Da im zusammenbebauten Zustand, über die Längsachse des Bioreaktors gesehen, jeweils alternierend die Septen 10 eines Bodenteils 4 des Grundkörpers 2 beziehungsweise 3 mit den Septen 10 des gegenüberliegenden Bodenteils 4 des Grundkörpers 3 beziehungsweise 2 angeordnet sind, sind die gebildeten Spalten 12 auch jeweils alternierend an dem Bodenteil 4 des einen Grundkörpers 2 beziehungsweise 3 und dem Bodenteil 4 des anderen Grundkörpers 3 beziehungsweise 2 angeordnet. Die dergestalt alternierend angeordneten Septen 10 bilden somit Unterkammern 18 dadurch, dass ein Septum 10 eines Grungkörpers 2 beziehungsweise 3 in die Kammer 11 des gegenüberliegenden Grundkörpers 3 beziehungsweise 2 hineinragt und so diese Kammer 11 in zwei Unterkammern 18 unterteilt. Das im Reaktor befindliche Medium wird entlang der Längsachse des Bioreaktors 1 mäanderförmig um die Septen 10 durch die Spalten 12 vom Bodenteil 4 des Grundkörpers 2 beziehungsweise 3 zum Bodenteil 4 des gegenüberliegenden Grundkörpers 3 beziehungsweise 2 geführt. Die Grundkörper werden im Tiefzieh- oder Spritzgießverfahren hergestellt.

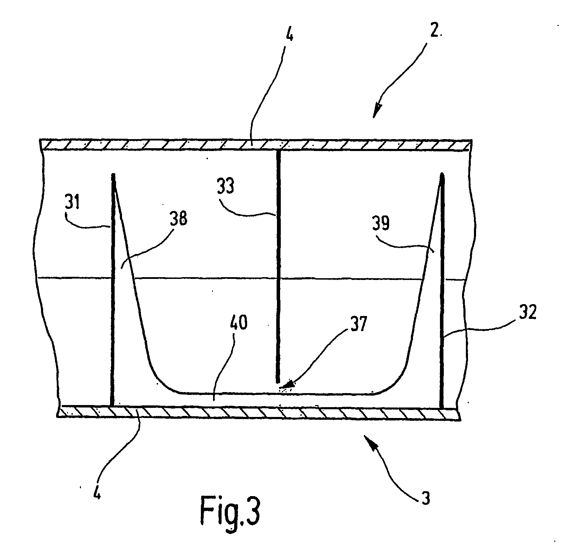

Bei der in Figur 3 ausschnittsweise vergrößert dargestellten Ausführungsform sind zwei zu dem Grundkörper 3 gehörige Septen 31 und 32 über einen Unterzug 35 miteinander verbunden. Der Unterzug 35 hat die Form eines U mit zwei Schenkeln 38 und 39, die durch eine Steg 40 miteinander verbunden sind. Der Schenkel 38 ist einstückig mit dem Septum 31.

Der Schenkel 39 ist einstückig mit dem Septum 32. Der Steg 40 ist einstückig mit dem Bodenteil 4 des Grundkörpers 3. Der Unterzug 35 hat die gleiche Dicke wie die Septen 31 und 32. Die Schenkel 38 und 39 sind an ihren freien Enden spitz zulaufend ausgebildet. Die Abmessungen des Stegs 40 sind so gewählt, dass zwischen der Spitze eines zwischen den Septen 31 und 32 angeordneten Septums 33, das zu dem Grundkörper 2 gehört, ein kleiner Spalt 37 verbleibt. Durch den Spalt 37 ist sichergestellt, dass das Septum 33 nicht an dem Steg 40 anliegt, was zu Beschädigungen führen könnte.

Der erfindungsgemäße Bioreaktor wird im Betrieb so aufgestellt, dass die Längsseitenteile 6 der Grundkörper 2 und 3 vertikal angeordnet sind. Demzufolge entspricht die Länge der Längsseitenteile 6 der Höhe des Bioreaktors, die beispielsweise etwa 2 Meter betragen kann. Die Länge der Querseitenteile 7 der Grundkörper 2 und 3 definiert die Breite des Bioreaktors, die beispielsweise etwa 1 Meter betragen kann. Die Tiefe des Bioreaktors kann beispielsweise 1,5 bis 5 cm betragen.

## Patentansprüche

1. Bioreaktor für die Kultivierung von Mikroorganismen, mit einem lichtdurchlässigen Gehäuse (2,3), das von durch Gaseintrag gebildeten Gasblasen in einer Strömungsrichtung durchströmt wird und in dem eine Strömungsleiteinrichtung (10) angeordnet ist, **dadurch gekennzeichnet, dass** die Strömungsleiteinrichtung mindestens zwei Septen (10) umfasst, die quer zur Strömungsrichtung angeordnet sind.

2. Bioreaktor nach Anspruch 1, **dadurch gekennzeichnet, dass** die Septen (10) so ausgebildet und in dem Gehäuse (2,3) angeordnet sind, dass sich zwischen den Septen (10) in dem Gehäuse (2,3) für die Gasblasen ein im Wesentlichen mäanderförmiger Strömungsweg ergibt und die Flüssigkeit zwischen den Septen (10) sich in Walzenform bewegt.

3. Bioreaktor nach einem der vorhergehenden Ansprüche, **dadurch gekennzeichnet, dass** die Septen (10) so ausgebildet und in dem Gehäuse (2,3) angeordnet sind, dass sie den durchströmten Querschnitt jeweils auf einen Spalt (12) begrenzen, wobei die von den Septen begrenzten Spalten (12) alternierend an gegenüberliegenden Seitenwänden (6) des Gehäuses (2,3) angeordnet sind.

4. Bioreaktor nach einem der vorhergehenden Ansprüche, **dadurch gekennzeichnet, dass** die Septen (10) durch mindestens ein Tragelement (2,3) miteinander verbunden sind.

5. Bioreaktor nach einem der Ansprüche 1 bis 4, **dadurch gekennzeichnet, dass** das Gehäuse zwei baugleiche Grundkörper (2,3) umfasst, wobei der Grundkörper (2,3) wannenförmig aus einem Bodenteil (4) und vier auf dem Bodenteil (4) angeordneten, eine lichte Tiefe T₁ aufweisenden Seitenteilen (5) aufgebaut ist, wobei die baugleichen Grundkörper (2,3) deckungsgleich aufeinander angeordnet sind.

6. Bioreaktor nach Anspruch 4, **dadurch gekennzeichnet, dass** die Strömungsleiteinrichtung mindestens ein Septum (10) umfasst, durch das zwei als Längsseitenteile (6) bezeichnete Seitenteile (5), die parallel gegenüberliegend im rechten Winkel zu dem Septum (10) angeordnet sind, so miteinander verbunden sind, dass in dem wannenförmigen Grundkörper (2,3) mehrere auf der dem zugehörigen Bodenteil (4) abgewandten Seite offene Kammern (11) gebildet werden, wobei das mindestens eine Septum (10) eine Septumtiefe (T₂) kleiner als die oder gleich der lichten Tiefe (T) des Reaktors (1) und größer als die halbe lichte Tiefe (T) des Reaktors (1) aufweist, wobei die Distanzen (D) zwischen den einzelnen Septen (10) gleich sind und die Distanz (D₁) zwischen einem als erstes Querseitenteil (13) bezeichneten Seitenteil (5) und einem darauf folgenden Septum (14) ungleich der Distanz (D₂) zwischen einem als zweites Querseitenteil (16) bezeichneten Seitenteil (5) und einem darauf folgenden Septum (15) oder einem ganzzeiligen Vielfachen davon ist, wobei die baugleichen Grundkörper mit ihren Kammeröffnungen (11) gegenüberliegend so aufeinander angeordnet sind, dass die Distanz (D₂) über der ersten Distanz (D₁) aufliegt.

7. Bioreaktor nach Anspruch 5 oder 6, **dadurch gekennzeichnet, dass** das lichtdurchlässige Material der Grundkörper (2,3) eine flexible Folie ist, vorzugsweise aus Kunststoff.

8. Bioreaktor nach Anspruch 6 oder 7, **dadurch gekennzeichnet, dass** der Grundkörper (2,3) in Längsrichtung mit mindestens einer Verstärkung (35) ausgestattet ist.

9. Bioreaktor nach Anspruch 8, **dadurch gekennzeichnet, dass** die Verstärkung von mehreren U-förmigen Unterzügen (35) gebildet wird, die jeweils mit zwei benachbarten Septen (31,32) und dazwischen mit dem Bodenteil (4) des zugehörigen Grundkörpers (3) verbunden sind, wobei das erste und das zweite Querseitenteil ebenfalls über jeweils einen U-förmigen Unterzug mit dem darauf folgenden Septum verbunden sind.

10. Bioreaktor nach einem der Ansprüche 6 bis 9, **dadurch gekennzeichnet, dass** die Wandstärke der Septen (10) zu dem Bodenteil des zugehörigen Grundkörpers hin zunimmt.

11. Bioreaktor nach einem der vorhergehenden Ansprüche, **dadurch gekennzeichnet, dass** seine beiden Grundkörper (2,3) an den zum Bodenteil (4) beabstandeten Kanten (8) seiner Seitenteile (5) einen im Wesentlichen rechtwinklig nach außen abstehenden Kragen (9) aufweisen.

12. Bioreaktor nach einem der Ansprüche 6 bis 11, **dadurch gekennzeichnet, dass** die Wandstärke der Seitenteile (5) des Grundkörpers (2) beziehungsweise (3) größer als die Wandstärke der Septen (10) des Grundkörpers (2,3) ist.

13. Bioreaktor nach einem der Ansprüche 11 oder 12, **dadurch gekennzeichnet, dass** die beiden Grundkörper (2,3) an dem jeweils nach außen abstehenden Kragen (9) der vom Bodenteil beabstandeten Kante(8) der Seitenteile (5) des Grundkörpers (2,3) miteinander verklebt oder mittels Ultraschall miteinander verschweißt sind.

14. Bioreaktor nach einem der vorhergehenden Ansprüche, **dadurch gekennzeichnet, dass** der von beiden Grundkörpern (2,3) gebildete Reaktorraum eine Oberflächenvergrößerung größer als eine geradflächige umhüllende Fläche seines Volumens aufweist.

15. Bioreaktor nach einem der vorhergehenden Ansprüche, **dadurch gekennzeichnet, dass** er Einrichtungen für eine turbulente Strömungsführung aufweist.

16. Bioreaktor nach einem der vorhergehenden Ansprüche, **dadurch gekennzeichnet, dass** er Elemente aufweist, die Licht von außen in den Reaktorraum leiten.

17. Bioreaktor nach einem der vorhergehenden Ansprüche, **dadurch gekennzeichnet, dass** er Anschlüsse und Anschlussleitungen für die Zu- und/oder Abfuhr von Gasen und/oder Flüssigkeiten aufweist.

18. Bioreaktor nach einem der vorhergehenden Ansprüche, **dadurch gekennzeichnet, dass** die Anschlussleitungen kühl- und/oder heizbar sind.

19. Bioreaktor nach einem der vorhergehenden Ansprüche, **dadurch gekennzeichnet**e dass er Bestandteil eines Systems mehrerer einzelner Bioreaktoren ist, die fluidseitig in Reihe geschaltet sind.

20. Verfahren zur Herstellung eines Bioreaktors, insbesondere gemäß der Ansprüche 1 bis 19, wobei in einem ersten Verfahrensschritt aus einem lichtdurchlässigen Material ein Strang hergestellt wird, der eine Vielzahl von direkt aufeinander folgenden, in Reihe angeordnete Grundkörpern (2,3) gemäß der vorhergehenden Ansprüche aufweist, wobei die Grundkörper (2,3) in der Orientierung (D₂) eines Grundkörpers (2) beziehungsweise (3) gefolgt von (D₁) eines Grundkörpers (2) beziehungsweise (3) hintereinander angeordnet sind, in einem zweiten Verfahrensschritt der Strang in die einzelnen Grundkörper (2,3) zerlegt wird und in einem dritten Verfahrensschritt jeweils zwei aufeinanderfolgende Grundkörper (2,3) durch Umklappen eines zweiten auf den ersten Grundkörper (2,3) in der in Anspruch 1 beschriebenen Weise aufeinander angeordnet werden.

21. Verfahren nach Anspruch 20, wobei die beiden Grundkörper (2,3) an dem jeweils nach außen abstehenden Kragen (9) der vom Bodenteil (4) beabstandeten Kante (8) der Seitenteile (5) des Grundkörpers (2) beziehungsweise (3) miteinander verklebt oder mittels Ultraschall miteinander verschweißt sind.

22. Verfahren nach einem der Ansprüche 20 oder 21, wobei der Strang im Spritzverfahren hergestellt wird.

23. Verfahren zur Kultivierung von Mikroorganismen, wobei diese in einen mit einer Flüssigkeit gefüllten Bioreaktor nach einem der Ansprüche 1 bis 19 eingebracht und dort unter geeigneten Bedingungen kultiviert werden.

24. Verfahren nach Anspruch 23, wobei die Kultivierung unter Gaseintrag erfolgt.

25. Verfahren nach einem der Ansprüche 23 oder 24, wobei die Kultivierung unter Zufuhr von CO₂ stattfindet.

26. Verfahren nach einem der Ansprüche 23 bis 25, wobei die Kultivierung unter Kühlung stattfindet.

## Claims

1. Bioreactor for cultivating microorganisms, with a light permeable housing (2,3), through which gas bubbles formed by gas introduction flow in a flow direction and in which a flow guide device (10) is arranged, **characterized in that** the flow guide device comprises at least two septa (10) arranged transversely to the flow direction.

2. Bioreactor according to Claim 1, **characterized in that** the septa (10) are constructed and arranged inside the housing (2,3) in such a way that an essentially meander-shaped flow path for the gas bubbles is obtained between the septa (10) inside the housing (2,3) and the fluid between the septa (10) moves in a revolving shape.

3. Bioreactor according to one of the previous claims, **characterized in that** the septa (10) are constructed and arranged in the housing (2,3) in such a way that they limit the flow cross-section in each case to only a gap (12), whereby the gaps (12) defined by the septa are arranged so as to alternate on opposing side walls (6) of the housing (2,3).

4. Bioreactor according to one of the previous claims, **characterized in that** the septa (10) are interconnected by at least one support element (2,3).

5. Bioreactor according to one of Claims 1 to 4, **characterized in that** the housing comprises two identically constructed base elements (2,3), whereby the base element (2,3) is constructed trough-like from a bottom part (4) and four side parts (5) arranged on the bottom part (4) and having an inside depth T₁, whereby the identically constructed base elements (2,3) are arranged on each other so as to exactly cover each other.

6. Bioreactor according to Claim 4, **characterized in that** the flow guide device comprises at least one septum (10), through which two side parts (5), called longitudinal side parts (6), which are arranged parallel, opposite from each other at a right angle to the septum (10), are interconnected in such a way that in the trough-like base element (2,3) several chambers (11) that are open on the side facing away from the associated bottom part (4) are formed, whereby the at least one septum (10) has a septum depth (T₂) that is smaller than or equal to the inside depth (T) of the reactor (1) and greater than half of the inside depth (T) of the reactor (1), whereby the distances (D) between the individual septa (10) are equal and the distance (D₁) between a side part (5) called the first transverse side part (13) and a consecutive septum (14) do not equal the distance (D₂) between a side part (5) called the second transverse side part (16) and a consecutive septum (15) or an integer multiple of it, whereby the identically constructed base elements with their chamber openings (11) are arranged opposite each other in such a way on top of each other that the distance (D₂) overlaps the first distance (D₁).

7. Bioreactor according to Claim 5 or 6, **characterized in that** the light-permeable material of the base elements (2,3) is a flexible foil, preferably of plastic.

8. Bioreactor according to Claim 6 or 7, **characterized in that** the base element (2,3) is provided in longitudinal direction with at least one reinforcement (35).

9. Bioreactor according to Claim 8, **characterized in that** the reinforcement is formed by several U-shaped bearing elements (35), which are individually connected with two adjoining septa (31,32) and in between with the bottom part (4) of the associated base element (3), whereby the first and second transverse side part each are also connected via a U-shaped bearing element with the following septum.

10. Bioreactor according to one of Claims 6 to 9, **characterized in that** the wall thickness of the septa (10) increases towards the bottom part of the associated base element.

11. Bioreactor according to one of the previous Claims, **characterized in that** both of its base elements (2, 3) are provided on the edges (8) of their side parts (5) that are at a distance to the bottom part (4) with a collar (9) projecting essentially at a right angle outward.

12. Bioreactor according to one of Claims 6 to 11, **characterized in that** the wall thickness of the side parts (5) of the base element (2) or (3) is greater than the wall thickness of the septa (10) of base element (2,3).

13. Bioreactor according to one of Claims 11 or 12, **characterized in that** the two base elements (2,3) are glued to each other or welded to each other via ultrasound at the respective outward-projecting collar (9) of the edge (8), located at a distance from the bottom part, of the side parts (5) of the base element (2,3).

14. Bioreactor according to one of the previous Claims, **characterized in that** the reactor space formed by both base elements (2,3) has a surface enlargement greater than a straight-surfaced enveloping surface of its volume.

15. Bioreactor according to one of the previous claims, **characterized in that** it has devices for a turbulent flow configuration.

16. Bioreactor according to one of the previous claims, **characterized in that** it has elements that guide the light from outside into the reactor space.

17. Bioreactor according to one of the previous claims, **characterized in that** it has connections and connecting lines for supplying and/or removing gases and/or fluids.

18. Bioreactor according to one of the previous claims, **characterized in that** the connecting lines can be cooled and/or heated.

19. Bioreactor according to one of the previous claims, **characterized in that** it is part of a system of several individual bioreactors that are arranged serially on the fluid side.

20. Method for producing a bioreactor, in particular according to Claims 1 to 19, wherein in a first step a strand from a light-permeable material is produced, said strand comprising a plurality of directly adjoining, serially arranged base elements (2,3) according to the preceding claims, whereby the base elements (2,3) are arranged behind each other in each case in orientation (D₂) of one base element (2) or (3) followed by (D₁) of another base element (2) or (3), wherein, in a second step, the strand is divided into the individual base elements (2,3), and wherein, in a third step, two each consecutive base elements (2,3) are arranged on top of each other by folding over a second base element onto a first base element (2,3) in the manner described in Claim 1.

21. Method according to Claim 20, wherein the two base elements (2,3) are glued to each other or welded to each other via ultrasound at the respective outward-projecting collar (9) of the edge (8), located at a distance from the bottom part (4), of the side parts (5) of the base element (2) or (3).

22. Method according to one of Claims 20 or 21, wherein the strand is produced by injection molding.

23. Method for cultivating microorganisms, wherein these are introduced into a bioreactor according to one of Claims 1 to 19 that has been filled with a fluid and are cultivated therein under suitable conditions.

24. Method according to Claim 23, wherein the cultivation is performed with introduction of gas.

25. Method according to one of Claims 23 or 24, wherein the cultivation is performed with addition of CO₂.

26. Method according to one of Claims 23 to 25, wherein the cultivation takes place with cooling.

## Revendications

1. Bioréacteur pour la culture de micro-organismes, comprenant un boîtier (2, 3) perméable à la lumière qui est traversé, dans un sens d'écoulement, par des bulles de gaz constituées par un apport de gaz et dans lequel est situé un dispositif de guidage de lécoulement (10), **caractérisé en ce que** le dispositif de guidage de l'écoulement comprend au moins deux cloisons (10) qui sont disposées transversalement par rapport au sens de l'écoulement.

2. Bioréacteur selon la revendication 1, **caractérisé en ce que** les cloisons (10) sont réalisées et disposées dans le boîtier (2, 3) de manière telle qu'il en résulte, pour les bulles de gaz, un chemin d'écoulement sensiblement en méandres entre les cloisons (10) dans le boîtier (2, 3) et que le liquide se déplace cylindriquement entre les cloisons (10).

3. Bioréacteur selon l'une des revendications précédentes, **caractérisé en ce que** les cloisons (10) sont réalisées et disposées dans le boîtier (2, 3) de manière telle qu'elles limitent la section traversée par l'écoulement à respectivement une fente (12), les fentes (12) limitées par les cloisons étant disposées en alternance sur des parois latérales (6) opposées du boîtier (2, 3).

4. Bioréacteur selon l'une des revendications précédentes, **caractérisé en ce que** les cloisons (10) sont reliées entre elles par au moins un élément porteur (2, 3).

5. Bioréacteur selon l'une des revendications 1 à 4, **caractérisé en ce que** le boîtier comprend deux corps de base (2, 3) de construction identique, le corps de base (2, 3) étant construit en forme de cuve à partir d'une partie de fond (4) et de quatre parties latérales (5) disposées sur la partie de fond (4) et présentant une profondeur intérieure T₁, les corps de base (2, 3) de construction identique étant placés l'un sur l'autre de manière à coïncider.

6. Bioréacteur selon la revendication 4, **caractérisé en ce que** le dispositif de guidage de l'écoulement comprend au moins une cloison (10) par laquelle deux parties latérales (5) appelées parties latérales longitudinales (6), lesquelles sont situées parallèlement à l'opposé l'une de l'autre à angle droit par rapport à la cloison (10), sont reliées entre elles de manière telle que sont constituées, dans le corps de base (2, 3) en forme de cuve, plusieurs chambres (11) ouvertes du côté opposé à la partie de fond (4) associée, l'au moins une cloison (10) présentant une profondeur de cloison (T₂) inférieure ou égale à la profondeur intérieure (T) du réacteur (1) et supérieure à la demi-profondeur intérieure (T) du réacteur (1), les distances (D) entre les cloisons (10) individuelles étant égales et la distance (D₁) entre une partie latérale (5) appelée première partie latérale transversale (13) et une cloison (14) suivante étant inégale à la distance (D₂) entre une partie latérale (5) appelée deuxième partie latérale transversale (16) et une cloison (15) suivante ou étant un multiple entier de celle-ci, les corps de base de construction identique, avec leurs ouvertures de chambres (11), étant situés l'un sur l'autre de façon opposée de manière telle que la distance (D₂) se trouve au-dessus de la première distance (D₁).

7. Bioréacteur selon la revendication 5 ou 6, **caractérisé en ce que** le matériau des corps de base (2, 3) perméable à la lumière est une feuille souple, de préférence en matière synthétique.

8. Bioréacteur selon la revendication 6 ou 7, **caractérisé en ce que** le corps de base (2, 3) est équipé d'au moins un renforcement (35) dans le sens longitudinal.

9. Bioréacteur selon la revendication 8, **caractérisé en ce que** le renforcement est constitué par plusieurs renforts (35) en forme de U qui sont respectivement assemblés à deux cloisons (31, 32) voisines et, entre celles-ci, à la partie de fond (4) du corps de base (3) associé, la première et la deuxième partie latérale transversale étant également assemblées à la cloison suivante respectivement par l'intermédiaire d'un renfort en forme de U.

10. Bioréacteur selon l'une des revendications 6 à 9, **caractérisé en ce que** l'épaisseur de paroi des cloisons (10) augmente en direction du la partie de fond du corps de base associé.

11. Bioréacteur selon l'une des revendications précédentes, **caractérisé en ce que** ses deux corps de base (2, 3) comportent, sur les bords (8) de ses parties latérales (5), lesquels sont à distance de la partie de fond (4), un rebord (9) tourné vers l'extérieur sensiblement à angle droit.

12. Bioréacteur selon l'une des revendications 6 à 11, **caractérisé en ce que** l'épaisseur de paroi des parties latérales (5) du corps de base (2) respectivement (3) est supérieure à l'épaisseur de paroi des cloisons (10) du corps de base (2,3).

13. Bioréacteur selon l'une des revendications 11 ou 12, **caractérisé en ce que** les deux corps de base (2, 3) sont collés ensemble ou soudés ensemble par ultrasons au rebord (9), respectivement tourné vers l'extérieur, du bord (8), situé à distance de la partie de fond, des parties latérales (5) du corps de base (2, 3).

14. Bioréacteur selon l'une des revendications précédentes, **caractérisé en ce que** l'espace de réacteur constitué par les deux corps de base (2, 3) présente une augmentation de surface supérieure à une surface enveloppante de surface droite de son volume.

15. Bioréacteur selon l'une des revendications précédentes, **caractérisé en ce qu'**il comporte des dispositifs pour un guidage turbulent de l'écoulement.

16. Bioréacteur selon l'une des revendications précédentes, **caractérisé en ce qu'**il comporte des éléments qui guident la lumière de l'extérieur vers l'espace de réacteur.

17. Bioréacteur selon l'une des revendications précédentes, **caractérisé en ce qu'**il comporte des raccordements et des conduites de raccordement pour l'amenée et/ou l'évacuation de gaz et/ou de liquides.

18. Bioréacteur selon l'une des revendications précédentes, **caractérisé en ce que** les conduites de raccordement peuvent être refroidies et/ou chauffées.

19. Bioréacteur selon l'une des revendications précédentes, **caractérisé en ce qu'**il fait partie intégrante d'un système de plusieurs bioréacteurs individuels qui sont montés en série côté fluide.

20. Procédé de fabrication d'un bioréacteur, en particulier selon les revendications 1 à 19, une barre étant fabriquée, dans une première étape, à partir d'un matériau perméable à la lumière, laquelle comporte un grand nombre de corps de base (2, 3) selon les revendications précédentes, disposés en série et se succédant directement, les corps de base (2,3) étant disposés les uns à la suite des autres dans l'orientation (D₂) d'un corps de base (2) respectivement (3), suivie de (D₁) d'un corps de base (2) respectivement (3), la barre étant, dans une deuxième étape, décomposée pour obtenir les corps de base (2, 3) individuels et respectivement deux corps de base (2, 3) successifs étant, dans une troisième étape, placés l'un sur l'autre par rabattement d'un deuxième sur le premier corps de base (2, 3) de la manière décrite dans la revendication 1.

21. Procédé selon la revendication 20, les deux corps de base (2, 3) sont collés ensemble ou soudés ensemble par ultrasons au rebord (9), respectivement tourné vers l'extérieur, du bord (8), situé à distance de la partie de fond (4), des parties latérales (5) du corps de base (2) respectivement (3).

22. Procédé selon l'une des revendications 20 ou 21, la barre étant fabriquée par injection.

23. Procédé pour la culture de micro-organismes, ceux-ci étant introduits dans un bioréacteur selon l'une des revendications 1 à 19 rempli d'un liquide et y étant cultivés dans des conditions appropriées.

24. Procédé selon la revendication 23, la culture se faisant sous apport de gaz.

25. Procédé selon l'une des revendications 23 ou 24, la culture se faisant sous amenée de CO₂.

26. Procédé selon l'une des revendications 23 à 25, la culture se faisant sous refroidissement.
